# EUROPEAN PATENT APPLICATION

(11) **EP 4 560 421 A1**
(43) Date of publication of application: **28.05.2025**
(21) Application number: 24213793.3
(22) Date of filing: 19.11.2024
(51) Int. Cl.: G05B 19/418, A61F 13/15, G05B 23/02

(54) **PRODUCTION LINE FOR ABSORBENT SANITARY ARTICLES**

(30) Priority: 23.11.2023 IT 202300024864
(71) Applicant: GDM S.p.A., 40133 Bologna (IT)
(72) Inventor: ZAVALLONI, Alessandro, I-40133 Bologna (IT); PALLAVERA, Massimo, I-40133 Bologna (IT); GIARDINI, Francesco, I-40133 Bologna (IT); PIGOLA, Luca, I- 40133 Bologna (IT)
(74) Representative: Porta & Consulenti Associati S.p.A.

(57) **Abstract**

Production line (100) for absorbent sanitary articles (2) and related control method, in which, in relation to a determined time period of operation of the production line (100), it is provided to:
- obtain discard event data indicative of discard events and stop event data indicative of stop events;
- process the discard event data in order to classify the discard events according to their incidence on the total number of absorbent sanitary articles discarded during said determined time period and select a limited number of discard events that had the greatest impact on the total number of discarded absorbent sanitary articles;
- process the stop event data obtained in order to classify the stop events according to their incidence on a total stop time of the production line during said determined time period and identify a limited number of stop events that had the greatest impact on the total stop time of the production line;
- identify discard root causes for the selected limited number of discard events as well as stop root causes for the selected limited number of stop events;
- define an action to be implemented with reference to at least one of the identified stop root causes and the identified discard root causes.

## Description

The invention relates to a production line for absorbent sanitary articles and a method for controlling the production of absorbent sanitary articles.

The absorbent sanitary articles are for example nappies for infants, sanitary towels or tampons for women, pads for people suffering from incontinence and the like.

Typically, such articles are produced in a production line characterized by high construction complexity, and at the same time the nappies themselves have high structural complexity. For this reason, production can be affected by elements that degrade production capacity.

As is well known, OEE (Overall Equipment Effectiveness) is a performance index used to measure the production capacity of a plant.

The OEE index is mainly derived from three elements: availability A, performance P and quality Q.

Availability A indicates the percentage of a plant's actual working time in relation to available time (e.g. in terms of daily shifts and working days or weeks).

Performance P indicates the percentage of parts actually produced compared to the parts theoretically achievable in the actual working time.

Quality Q indicates the percentage ratio of conforming parts to the total number of parts actually produced.

The OEE index is generally obtained by multiplying the three percentages indicated above (OEE=A*P*Q)

In this context, the Applicant perceived the need to provide an automated control method of a production line for absorbent sanitary articles that enables its production efficiency management to be improved.

The present invention therefore relates, in a first aspect thereof, to a production line of absorbent sanitary articles.

Preferably, the production line comprises a working area configured to carry out respective production operations on the sanitary articles being processed along the production line and an automated production line control and monitoring system.

Preferably, the control and monitoring system comprises control devices operatively associated with the working area and a central control unit operatively connected to the control devices.

Preferably, the central control unit comprises a processor configured, in relation to a determined time period of operation of the production line, to obtain discard event data indicative of discard events that led to discarding absorbent sanitary articles during the determined time period and, for each discard event, specific discard data indicative of the number of absorbent sanitary articles discarded in association with the discard event.

Preferably, the processor is configured, in relation to the determined time period, to obtain stop event data indicative of stop events that led to stop the production line during the determined time period and, for each stop event, specific stop data indicative of a stop time of the production line associated with the stop event.

Preferably, the processor is configured to process the discard event data and the specific discard data obtained in order to classify the discard events according to their incidence on the total number of absorbent sanitary articles discarded during said determined time period and select a limited number of discard events that had the greatest impact on the total number of discarded absorbent sanitary articles.

Preferably, the processor is configured to process the stop event data and the specific stop data obtained in order to classify the stop events according to their incidence on a total stop time of the production line during said determined time period and select a limited number of stop events that had the greatest impact on the total stop time of the production line.

Preferably, the processor is configured to identify discard root causes for the selected limited number of discard events.

Preferably, the processor is configured to identify stop root causes for the selected limited number of stop events.

Preferably, the processor is configured to define an action to be implemented with reference to at least one of the identified stop root causes and the identified discard root causes.

The present invention thus relates, in a second aspect thereof, to a method for controlling the production of absorbent sanitary articles in a production line.

Preferably, in relation to a determined time period of operation of the production line, it is provided to obtain discard event data indicative of discard events that led to discarding absorbent sanitary articles during the determined time period and, for each discard event, specific discard data indicative of the number of absorbent sanitary articles discarded in association with the discard event.

Preferably, in relation to said determined time period of operation, it is provided to obtain stop event data indicative of stop events that led to stop the production line during the determined time period and, for each stop event, specific stop data indicative of a stop time of the production line associated with the stop event.

Preferably, it is provided to process the discard event data and the specific discard data obtained in order to classify the discard events according to their incidence on the total number of absorbent sanitary articles discarded during said determined time period and select a limited number of discard events that had the greatest impact on the total number of discarded absorbent sanitary articles.

Preferably, it is provided to process the stop event data and the specific stop data obtained in order to classify the stop events according to their incidence on a total stop time of the production line during said determined time period and select a limited number of stop events that had the greatest impact on the total stop time of the production line.

Preferably, it is provided to identify discard root causes for the selected limited number of discard events.

Preferably, it is provided to identify stop root causes for the selected limited number of stop events.

Preferably, it is provided to define an action to be implemented with reference to at least one of the identified stop root causes and the identified discard root causes.

According to the invention, it is thus provided to monitor discard events and stop events during a determined time period, to classify them according to their impact on the total number of discarded sanitary articles and, respectively, on the total stop time of the production line, to identify discard root causes and stop root causes only for a limited number of the classified discard events and stop events, and to define an action to be implemented to remedy the discard root causes and the stop root causes thus identified.

Among the discard events and stop events that can contribute to degradation of the OEE index of the production line, it is thus provided to identify the discard root causes and the stop root causes and, subsequently, the corrective actions to be implemented in order to remedy these causes and thus improve the OEE, only for those events that may have had the greatest influence on degradation of the OEE index.

Given the high construction complexity of the production line and the high structural complexity of the articles themselves, a discard event or stop event could be generated by several possible root causes. Tracing back, from among a plurality of possible root causes, to the root cause that is currently responsible for the discard event or for the stop event can be a non-immediate and complex operation.

Limiting the operation of identification of root causes and the corrective actions to be implemented to the limited number of stop events and discard events identified as described above, advantageously allows to manage the production effectiveness of the production line in an efficient and automated manner.

"Absorbent sanitary article being processed" or "article being processed" indicates a semi-processed absorbent sanitary article at any step of the relative production process within a production line, including individual materials and individual elements of formation of the absorbent sanitary article.

The term "finished absorbent sanitary article" or "finished article" indicates a finished absorbent sanitary article obtained at the end of the production process.

"Absorbent sanitary article" or "article" indicates a finished absorbent sanitary article or an absorbent sanitary article being processed.

"Malfunctioning" referring to a production line indicates malfunctioning that may concern any operating device in the production line, including both a production device that is configured to perform a respective production operation on the absorbent sanitary articles being processed along the production line and an accessory device with respect to production, such as a device adapted to maintain adequate boundary conditions within the plant in which the production line is housed, such as for example a device adapted to maintain a certain level of temperature and/or humidity within the plant.

Such malfunctioning could generate a defect in the absorbent sanitary articles being processed along a production line and/or in finished absorbent sanitary articles in output from the production line and/or cause a stop of the line or of the malfunctioning production device.

"Defect" indicates an imperfection that is present or may occur in an absorbent sanitary article with respect to a predefined standard.

"Direction of advancement" referring to an article within a production line indicates a direction of movement of that article within that line. For example, in the case of an article moved by transport members (comprising, for example, a conveyor belt) it indicates a direction of movement of said transport members (in the example, of said conveyor belt).

The present invention may have, in the aspects discussed above, at least one of the preferred features disclosed below. These features may therefore be present individually or in combination with each other, unless expressly stated otherwise.

Preferably, the working area comprises production devices configured to perform respective production operations on the absorbent sanitary articles being processed along the production line.

Preferably, the production devices comprise at least one of:
- transport members adapted to support and move the absorbent sanitary articles being processed along the line;
- feeding devices adapted to feed elements of the absorbent sanitary articles being processed (for example in the form of strips) and to place them, at least partially mutually overlapping, on a supporting surface of the transport members;
- retaining members configured to retain in position the elements placed on the supporting surface of the transport members, preferably comprising suction devices active on retaining holes formed on the supporting surface of the transport members;
- at least one fixing device adapted to fix the elements of the absorbent sanitary articles being processed together (for example by welding and/or gluing);
- at least one cutting device adapted to cut elements of the absorbent sanitary articles being processed and/or a continuous strip of the absorbent sanitary articles being processed joined together, resulting from the production process, into individual articles.

Preferably the working area comprises accessory devices with respect to the production of articles.

For example, an accessory device could be a device adapted to maintain adequate boundary conditions within the plant in which the production line is housed, such as for example a device adapted to maintain a certain level of temperature and/or humidity within the plant.

Preferably, the processor is configured to automatically implement the defined action, said action comprising the automatic emission of a predetermined signal for an operator and/or a predetermined control signal for control devices.

Preferably, said control devices comprise actuators.

Preferably, the processor is configured to automatically convert the defined action to be taken into a control signal for the actuators.

The defined action to be taken may concern one or more of the production devices and/or other accessory devices, which are involved in the discard/stop root cause identified.

Preferably the actuators are operatively associated with said control unit.

Preferably, the actuators are operatively associated with at least one of the production and accessory devices.

Preferably, the actuators are configured to intervene on the operation of at least one of the production devices and accessory devices based on the control signal coming from the processor.

Preferably, the control devices comprise sensors and the discard event data, stop event data, specific discard data and specific stop data are obtained by the processor at least in part by processing data from said sensors.

Preferably, the sensors comprise at least one of: temperature sensor; pressure sensor; vibration sensor; position sensor; weight sensor; quantity sensor; flow sensor; vacuum level sensor; air jet status sensor; optical sensor; photoelectric sensor; proximity sensor; contour sensor; displacement sensor; tension sensor; speed sensor; torque sensor; electric current sensor.

Preferably, the sensors are configured to detect current values of operating parameters that are indicative of the current operation of at least one of the production devices and accessory devices of the production line.

Preferably, the sensors are configured to send an alarm or warning signal and/or to trigger a (preferably automatic) stop of the production line or of a production device or of an accessory device in the presence of at least one abnormal operating parameter whose current value does not comply with a respective reference value.

Preferably, the operating parameters detected by the plurality of sensors comprise at least one of: temperature; pressure; vibration; position of objects; weight; quantity; vacuum level; status of air jets; impact energy level of the cutting device; level of cleanliness; presence or quantity of material at a predetermined position; feeding tension of the elements of the absorbent sanitary articles being processed; speed; torque (e.g. of a motor) and absorbed current (e.g. by a motor).

In a preferred embodiment, the control devices comprise at least one inspection device configured to acquire article data directly correlated to the absorbent sanitary articles being processed along the production line and/or to the finished absorbent sanitary articles, in output from the line.

The article data can relate, for example, to the appearance, shape, weight, positioning, dimensions, contours of the articles being processed along the production line and/or to the finished absorbent sanitary articles, in output from the line.

Said at least one inspection device may, for example, be selected from: an image acquisition device, a weight sensor, a proximity sensor, a photoelectric sensor, a contour sensor, a displacement sensor, a position sensor and a quantity sensor.

Preferably, the processor is configured to detect any defects on the absorbent sanitary articles being processed along the production line and/or on the finished absorbent sanitary articles in output from the line, by processing the article data acquired by said at least one inspection device.

In the event of detecting defects, the processor is configured to collect and store (e.g. in a memory) data on each type of defect detected and the items rejected due to the identification of each type of defect.

Preferably, discard event data and specific discard data are obtained by the processor at least in part by processing data from said at least one inspection device.

Preferably, the control method is a computer-implemented method.

Preferably, the control method provides for the automatic implementation of the defined action, said action comprising the automatic emission of a predetermined signal for an operator and/or a control signal for a production line control device.

Preferably, the stop root causes and the discard root causes are identified by means of decision algorithms based on at least one of the following pieces of information: known and/or self-learned data relating to a correlation between the discard events and a plurality of possible discard root causes; known and/or self-learned data relating to a correlation between the stop events and a plurality of possible stop root causes; current and/or historical information automatically provided by a control and monitoring system of the production line in association with the discard events and/or the stop events; current and/or historical information provided by an operator in association with the discard events and/or the stop events; known and/or self-learned logic rules.

Preferably, said decision algorithms are implemented by means of artificial intelligence, expert system or preset and defined logics and correlations.

Preferably, said decision algorithms are assisted by special machine learning and/or statistical algorithms.

Preferably, the action to be implemented is defined (by means of the decision algorithms) to remedy at least one of the identified stop root causes and the identified discard root causes.

Preferably, the discard root causes comprise at least one discard root cause selected from: malfunctioning of a production device or other accessory device of the production line, material exhaustion, defective material, stop of a workstation (e.g. packaging machine) downstream of the production line, non-routine maintenance of the production line, absence of workforce.

Preferably, the stop root causes comprise at least one stop root cause selected from: malfunctioning of a production device or other accessory device of the production line, material exhaustion, defective material, stop of a workstation (e.g. packaging machine) downstream of the production line, non-routine maintenance of the production line, absence of workforce.

Preferably, among the classified discard events, the limited number of discard events is selected by identifying the discard events that impacted for at least 80% on the total number of the discarded absorbent sanitary articles.

Preferably, from among the classified stop events, the limited number of stop events is selected by identifying the stop events that impacted for at least 80% on the total stop time of the production line.

Preferably, the control method also includes the step of obtaining, in relation to said determined time period, availability data A indicative of an actual operation time of the production line with respect to a theoretical operation time, performance data P indicative of a quantity of absorbent sanitary articles actually produced with respect to a theoretical expected quantity for the actual operation time of the production line and quality data Q relating to a quantity of compliant absorbent sanitary articles with respect to the quantity of absorbent sanitary articles actually produced.

Preferably, the control method also comprises the step of determining an OEE index of the production line on the basis of the availability data A, the performance data P and the quality data Q obtained.

Preferably, the OEE index is determined in relation to said determined time period before the implementation of the defined action. Preferably, the OEE index is determined for a further determined time period after the implementation of the defined action. Preferably, it is provided to compare the OEE index determined before and after the implementation of the defined action in order to verify its effectiveness on the OEE index.

Preferably, the availability data A, performance data P and quality data Q are obtained at least in part by processing data from the automated control and monitoring system of the production line.

Preferably, the availability data A, performance data P and quality data Q are obtained at least in part by processing data from said sensors and/or said at least one inspection device.

Preferably, the discard event data, stop event data, specific discard data, specific stop data, the total number of discarded absorbent sanitary articles and the total stop time of the production line are obtained at least in part by processing data from the automated control and monitoring system of the production line.

Preferably, the discard event data, stop event data, specific discard data, specific stop data, the total number of discarded absorbent sanitary articles and the total stop time of the production line are obtained at least in part by processing data from said sensors and/or said at least one inspection device.

Preferably, the article comprises a plurality of elements.

Preferably, said elements are at least partly strip-shaped.

The elements can be distinguishable into base elements and additional elements.

The base elements preferably comprise a first sheet of permeable material and a second sheet of impermeable material, which are intended to define, respectively, the inner face and the outer face of the article.

The first sheet and the second sheet are typically mutually overlapping and between them there is interposed a third main element constituted by an absorbent padding.

The additional elements, which may vary in number and shape, preferably comprise front and/or rear flaps, acquisition and distribution layers or reinforcement layers or other discrete elements typically provided in the absorbent sanitary articles between the first sheet and the second sheet.

Preferably, within the production line, the absorbent sanitary articles being processed can be moved along a direction of advancement.

Preferably, the transport members are adapted to support and move the absorbent sanitary articles being processed along said direction of advancement.

In one embodiment, the transport members comprise at least one conveyor belt.

Preferably, the conveyor belt is movable along the direction of advancement for the movement of the articles being processed within the production line.

The conveyor belt may have an at least partly flat and/or curved supporting surface.

The direction of advancement may be at least partly straight and/or curved.

Further features and advantages of the present invention will become clearer from the following detailed disclosure of a preferred embodiment thereof, made with reference to the appended drawings and provided by way of indicative and non-limiting example, in which:
- Figure 1 schematically shows a production line of absorbent sanitary articles according to an embodiment of the invention;
- Figure 2 represents, in plan, a schematic example of an absorbent sanitary article that can be made by the line in Figure 1;
- Figure 3 shows an example of the production line of absorbent sanitary articles in Figure 1.

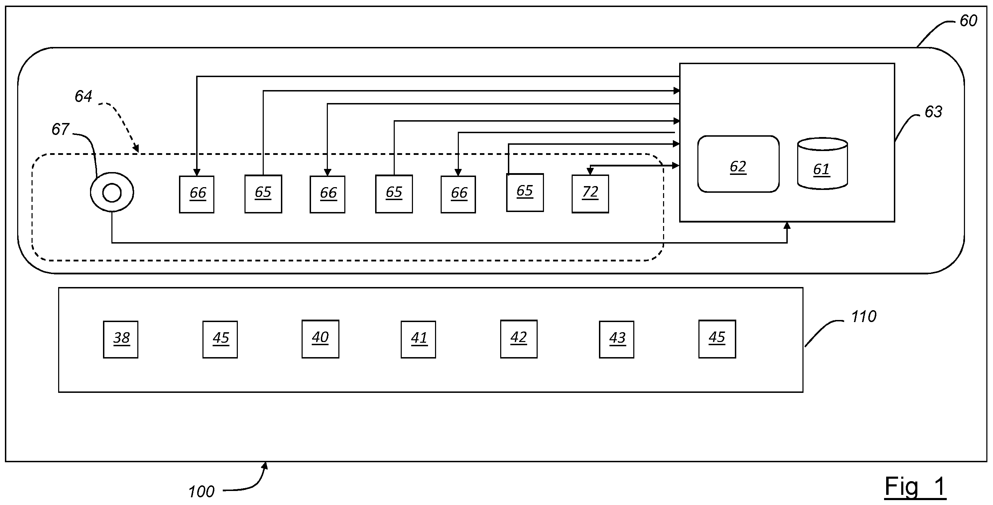

Figure 1 schematically shows a production line 100 of absorbent sanitary articles 2 according to an embodiment of the invention.

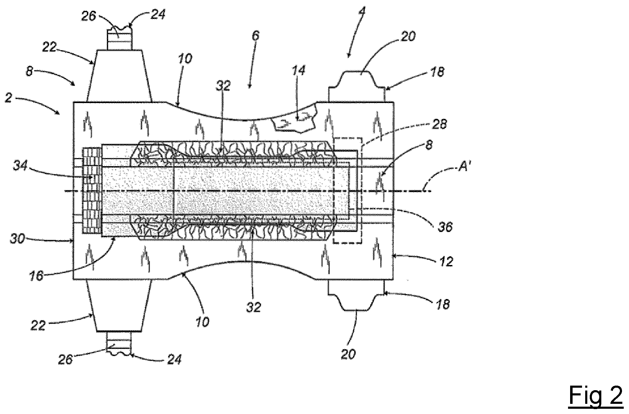

With reference to Figure 2, the absorbent sanitary articles 2 have a substantially rectangular shape. In the specific example the articles 2 are nappies for infants, however, in the spirit of the present invention and according to variants immediately deducible from the context and from what is disclosed below, the articles 2 may be sanitary towels for women, pads for senile incontinence or the like.

The articles 2 comprise, in alignment according to a longitudinal axis thereof, indicated with A', a front portion 4, wearable on the front part of the body of the user, a rear portion 8, wearable on the rear part of the body of the user, and a central portion 6, arranged between the front part and the rear part and wearable between the legs of the user. At the central portion 6 there is provided a recess 10, or leg line, defined by two arcuate sections that are symmetrical with respect to the axis A'.

The absorbent sanitary articles 2 are formed by a plurality of elements, distinguishable into base elements and additional elements. In particular, the base elements are assembled together in such a way as to define a base article, whose elements are arranged according to a preset and defined scheme. Similarly, the additional elements are applied on the base article during and/or after the definition thereof, according to a preset and defined scheme, so as to realize a finished article 2.

The base elements are constituted by a first sheet 12 of permeable material (non-woven fabric) and a second sheet 14 of impermeable material, which are intended to define, respectively, the inner face and the outer face of the article 2.

The sheets 12 and 14 are mutually overlapping and between them there is interposed a third main element constituted by an absorbent padding 16. The padding 16 is formed by cellulose fibre and superabsorbent polymer (SAP) granules dispersed therein.

The additional elements, which may vary in number and shape, are disclosed below with reference to the absorbent sanitary article 2 illustrated in Figure 2.

18 indicates two first shaped wings, each having a respective lobe 20, fixed to the inner face of the second impermeable sheet 14 and extending transversely to the axis A' from the front portion 4 of the article 2, and 22 indicates two shaped closing wings, parallel to the first wings 18, extending from the rear portion 8 of the article 2.

To each of the second closing wings 22 a third flap 24 is applied, provided with an adhesive strip 26, with a course parallel to the axis A', intended to adhere, in use, to a corresponding front strip 28 applied to the front portion 4 of the outer face of the second sheet 14. The wings 22 provided with the flaps 24 constitute, together with the front strip 28, means for closing the article 2.

The adhesive strip 26 can be replaced, according to a variant, with a Velcro^{®} strip.

To the first sheet 12 of permeable material, two strips 30 of impermeable material are laterally welded, for thickening and expanding the longitudinal edges thereof. The strips 30 are provided at their intermediate section with an elasticized portion 32.

A further additional component is constituted by an elastic band 34 applied, transversely to the axis A', to the inner face of the second sheet 14 at the rear portion 8 of the article 2.

At the inner face of the first sheet 12 of permeable material, in contact with the absorbent padding 16 and welded along the contour of the latter, there is provided a sheet 36 of absorbent material, defined as "acquisition layer", having the function of making uniform the absorbent power of the surface of the absorbent padding 16.

With reference to Figure 1, the production line 100 comprises a working area 110 and an automated control and monitoring system 60 of the production line 100.

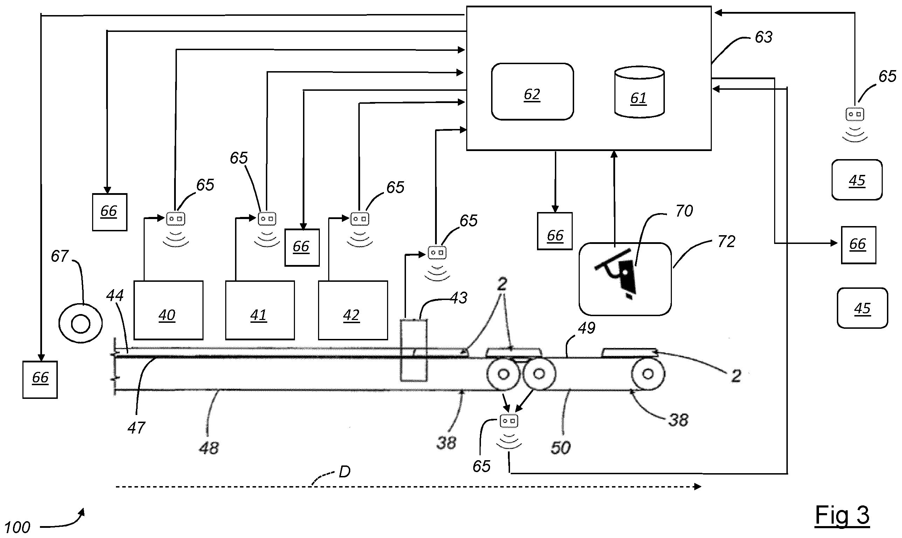

As described in more detail with reference to the example in Figure 3, the working area 110 comprises both production devices 38, 40, 41, 42, 43 and accessory devices 45 with respect to production, such as, for example, devices for maintaining adequate boundary conditions within the plant in which the production line 100 is housed.

For example, the accessory devices 45 may comprise equipment adapted to maintain a certain level of temperature and/or humidity within the plant.

The production devices 38, 40, 41, 42, 43 are configured to carry out respective production operations of the absorbent sanitary articles 2.

Such operations may comprise, for example, support and movement; feeding material constituting the base elements and the additional elements and/or the elements themselves; cutting the materials to make the individual elements or to make shapings to the already applied elements; placing the base elements and the additional elements in partial or total mutual overlapping; fixing the various base and additional elements by gluing and/or welding; cutting a continuous strip of the absorbent sanitary articles 2 being processed joined together, resulting from the production process, into individual articles, and the like.

The automated control and monitoring system 60 comprises control devices 64 located within the production line 100 and a central control unit 63.

The central control unit 63 comprises a processor 62 and a memory 61.

Although a single processor 62 and a single memory 61 are indicatively shown in Figure 1, it is clear that the central control unit 63 may be a complex system comprising a network of local and/or remote memories and local and/or remote processors suitably interconnected via wireless, wired or mixed connections.

The control devices 64 comprise actuators 66 and sensors 65 that are located within the production line 100 depending on the production requirements and on the configuration and structure of the production line 100.

Although 3 actuators 66 and 3 sensors 65 are indicatively shown in Figure 1, it is clear that a higher or lower number of actuators 66 and sensors 65 can be envisaged depending on production requirements and the configuration and structure of the production line 100.

In a preferred embodiment, the control devices 64 also comprise at least one inspection device 72 configured to acquire article data directly correlated to the absorbent sanitary articles 2 being processed along the production line 100 and/or to the finished absorbent sanitary articles, in output from the production line 100.

The article data can relate, for example, to the appearance, shape, weight, positioning, dimensions, contours of the articles 2 being processed along the production line and/or to the finished absorbent sanitary articles 2, in output from the production line 100.

Said at least one inspection device 72 may, for example, be selected from: an image acquisition device, a weight sensor, a proximity sensor, a photoelectric sensor, a contour sensor, a displacement sensor, a position sensor and a quantity sensor.

Preferably, the processor 62 is configured to detect any further defects on the absorbent sanitary articles 2 being processed along the production line 100 and/or on the finished sanitary towels 2 in output from the production line 100, by processing the article data acquired by said at least one inspection device 72. The processor 62 is configured to carry out defect detection according to techniques known in the art, hence these will not be described in further detail below.

The processor 62 is also configured to collect and store in the memory 61 data relating to defects detected and to the articles 2 discarded due to the detection of such defects.

Preferably, the control devices 64 also comprise at least one emergency stop device 67 (e.g. in the form of a pushbutton) configured to allow an operator to manually stop an individual production device 38, 40, 41, 42, 43 or the entire production line 100 if necessary (e.g. in case of detection of malfunctions or in dangerous situations).

Preferably, each time an emergency stop is made, the control system 60 is configured to allow the operator to enter notes indicating the stop event that led to the stop. For example, the stop event may be related to the unwanted presence of glue on the conveyor belt 48, 50 or to a material flying off the conveyor belt 48, 50. These notes, together with specific stop data indicative of the time the production line 100 is kept stopped, are stored in the memory 61. These notes can be entered by the operator by means of a suitable local graphic interface (not illustrated) at the emergency stop device 67 or at the individual production device 38, 40, 41, 42, 43 or by means of a central graphic interface (not illustrated) at the control unit 63.

The control unit 63 is operatively connected (via a suitable wired and/or wireless connection) to the sensors 65, the actuators 66, the inspection device 72 and the emergency stop device 67.

As described in more detail with reference to the example of Figure 3, by means of the sensors 65 and of the inspection device 72, the automated control and monitoring system 60 is adapted to monitor the operation of the production line 100 and, in particular, of the production devices 38, 40, 41, 42, 43 and of the accessory devices 45, to emit appropriate alarm signals or warning signals in the event of anomalies and, if necessary, to carry out interventions on the production devices 38, 40, 41, 42, 43 and/or on the accessory devices 45 by means of the actuators 66.

In the example illustrated in Figure 3, the production devices located in the working area (not shown in Figure 3) comprise conveying members 38 adapted to support and move, along an advancement direction D, the absorbent sanitary articles 2 being processed along the line 100.

In the illustrated embodiment, the direction of advancement D is straight.

The transport members 38 may comprise a plurality of conveyor belts. In particular, in Figure 3 there are indicated a first conveyor belt 48 with a respective supporting surface 47 and a second conveyor belt 50 with a respective supporting surface 49.

In the illustrated embodiment, the production devices also comprise feeding devices (indicatively and schematically illustrated in the figure with a single block 40), adapted to feed material constituting the base elements and the additional elements of the articles 2 being processed and/or the elements themselves and to place them, at least partially mutually overlapping, on a supporting surface 47 of the transport members 38.

For example, in the embodiment illustrated in Figure 3, the supporting surfaces 47 and 49 are flat.

In the embodiment illustrated in Figure 3, the production devices also comprise retaining members (indicatively and schematically illustrated in the figure with a single block 41) configured to retain in position the elements/materials placed on the supporting surface 47 of the first conveyor belt 48 and to retain in position the finished articles 2 on the transport surface 49 of the second conveyor belt 50. For example, the retaining members 41 comprise suction devices (not illustrated) active on retaining holes (not illustrated) formed on at least part of the supporting surface 47 of the first conveyor belt 48 and on at least part of the transport surface 49 of the second conveyor belt 50.

In the embodiment illustrated in Figure 3, the production devices also comprise at least one fixing device (indicatively and schematically illustrated in the figure with a single block 42) adapted to fix the base and additional elements together by gluing and/or welding.

Said at least one fixing device 42 may comprise an ultrasonic welding device.

In an alternative embodiment, said at least one fixing device 42 may comprise a thermo-mechanical welding device.

In addition or alternatively, said at least one fixing device 42 may comprise a gluing device comprising a glue dispensing device associated with a tank (not illustrated) for the glue. In one embodiment, said glue dispensing device may therein comprise a small secondary glue tank adapted to withdraw the glue, by means of a suitable withdrawal mechanism, from an external primary glue tank.

In the embodiment illustrated in Figure 3, the production devices also comprise at least one cutting device (indicatively and schematically illustrated in the figure with a single block 43) adapted to perform the aforesaid cutting operations. In particular, Figure 3 depicts a device adapted to make cuts or transverse weakening lines (with respect to the direction of advancement D) of the continuous strip 44 of absorbent sanitary articles being processed 2 in such a way as to be able to subsequently separate or make the absorbent sanitary articles 2 easily separable.

One or more of the production devices 40, 41, 42, 43 may be made by an individual device or by separate devices. Such an individual device or such separate devices may comprise one or more forming drums (not illustrated) which are rotatable about respective axes of rotation. In such a case, at least one between the first conveyor belt 48 and the second conveyor belt 50 may be wholly or partly replaced by transport surfaces of such forming drums.

The supporting surfaces 47 and 49 of the transport members 38 may thus be at least partly flat and/or curved. Furthermore the direction of advancement D may be at least partly straight and/or curved.

In Figure 3, the actuators 66 can be operatively associated at least in part with production devices 40, 41, 42, 43, 38 and/or accessory devices 45.

The actuators 66 may, for example, be adapted to regulate the passage of the air at the suction devices of the retaining members 41 and may comprise shutters whose opening/closing level can be controlled automatically.

Additionally or alternatively, the actuators 46 may comprise, for example, adjustable valves and/or pressure regulators and/or temperature regulators.

In Figure 3, the sensors 65 are operatively associated with the production devices 40, 41, 42, 43, 38 and accessory devices 45. In particular, the sensors 65 are configured to detect current values of operating parameters of the respective production devices 40, 41, 42, 43, 38 and accessory devices 45.

The operating parameters detected by the sensors 65 associated with the production devices 40, 41, 42, 43, 38 can be related to the process of feeding, positioning, assembling, cutting of the various constituent parts of the article 2 and/or their shape.

For example, the sensors 65 may comprise one or more temperature sensors, for example for measuring the temperature of the glue contained in the tank (not illustrated) associated with said at least one fixing device 42; one or more pressure sensors, for example adapted to measure a vacuum level created by the suction devices of the retaining members 41 and/or the pressure of the glue dispensed by said at least one fixing device 42; one or more vibration sensors such as, for example, an accelerometer adapted to measure, for example, the vibrations of a piece (such as, for example, the supporting surface 47 of the first conveyor belt 48) of a production device 38, 40, 41, 42, 43; one or more position sensors, for example adapted to measure the position of an object with respect to a reference or the mutual position of objects at one or more of the production devices 38, 40, 41, 42, 43. For example, the position sensors may be adapted to measure the position of a roller (not shown) of the feeding devices 40 with respect to an application point and/or the opening/closing of a shutter of the suction devices of the retaining members 41. The sensors 65 may further comprise one or more weight sensors (e.g. load cells), for example adapted to control the weight of the glue contained in the tank associated with said at least one fixing device 42 or the presence/absence/quantity of material at a predetermined position; one or more flow sensors such as, for example, a flow meter, for example adapted to measure the quantity of glue dispensed by said at least one fixing device 42; one or more air jet status sensors, for example adapted to detect the status (active/inactive/intensity) of the air jets created by the suction devices of the retaining members 41 (for example by measuring the pressure of the air upstream by the suction devices of the retaining members 41); one or more optical sensors, for example adapted to detect the level of cleanliness at a production device 38, 40, 41, 42, 43 or the presence/absence (and thus the possibly breakage) of a material/element fed by a feeding device 40; one or more force sensors (for example load cells), for example adapted to detect the feeding tension of the elements/materials of the absorbent sanitary articles fed by the feeding devices 40 and/or to verify whether compression members/rollers at the feeding devices 40 are operating correctly. Load cells can, for example, be used also/or to detect the impact energy level of said at least one cutting device 43. In addition or alternatively, the sensors 65 may comprise one or more sensors adapted to detect operating parameters of one or more motors present at least at one production device 38, 40, 41, 42, 43, such as torque, temperature and absorbed current.

The current values of the operating parameters measured by the sensors 65 are continuously compared with respective predetermined reference values.

When at least one of the detected current values does not comply with the respective predetermined reference value, the respective operating parameter is labelled as an abnormal operating parameter. In addition, a warning or alarm signal is preferably emitted. The warning or alarm signal can be given in audible and/or visual form (e.g. it can be emitted by a flashing acoustic device or displayed on a screen).

In an embodiment, the aforementioned operations of comparison and warning or alarm signal emission can be carried out locally by the sensors 65. In this case, the warning or alarm signal can be transmitted from the sensors 65 to the central control unit 63 which, as described in more detail below, can store it in the memory 61 and use it for analysis by the processor 62.

Alternatively, the aforementioned comparison and warning or alarm signal emission operations can be carried out by the central unit 63. In the latter case, the sensors 65 will be configured to send the current values of the detected operating parameters to the central unit 63, and the central unit will be able to store the values received from the sensors 65 together with the predetermined reference values in the memory 61.

In any case, when an abnormal operating parameter is detected and an alarm or warning signal is emitted, the control unit 63 is configured to keep track of it in the memory 61, possibly with the addition of some notes entered manually by an operator via an appropriate user interface (not shown) provided in the control unit 63.

When, following the detection of an abnormal operating parameter, the production line 100 is stopped, the processor 62 is preferably configured to collect and store in the memory 61 data relating to the stop event and to the stop time associated with that event.

In the embodiment illustrated in Figure 3, the inspection device 72 comprises an image acquisition device 70 (e.g., a camera) configured to acquire images of the finished absorbent sanitary articles 2, in output from the line 100.

The image acquisition device 70 may be arranged facing the second conveyor belt 50, as illustrated in Figure 3, to detect at least one image of each finished article 2, i.e. already separated from the continuous strip 44 of articles 2 joined together, or, according to a variant not illustrated, the image acquisition device 70 may be arranged facing the first conveyor belt 48 to detect at least one image of each article 2 when it has yet to be separated from the continuous strip 44 of articles 2 joined together. In both cases, the image acquisition device 70 is arranged at the output of the production line 100 to detect at least one image of each article 2 already defined relatively to the composition and assembly of the base and additional elements thereof.

Although not illustrated in Figure 3, alternatively or additionally, the inspection device 72 may comprise one or more image acquisition devices or, in general, article data acquisition devices located along the production line 100, for example in the vicinity of one or more of the production devices 38, 40, 41, 42, 43.

The control unit 63 is operatively connected (by means of a suitable wired and/or wireless connection) to the sensors 65, the actuators 66 and the image acquisition device 70.

In the embodiment of Figure 3, the processor 62 is preferably configured to detect any defects present in the finished absorbent sanitary articles 2 in output from the line by processing the images acquired by the image acquisition device 70 using suitable machine vision algorithms known in the art.

Typical defects that can be detected by said image processing may comprise: wrong alignment of the absorbent padding 16, wrong orientation of the front strip 28, wrong positioning of the closing wings 22 and wrong execution of the cut of the recess 10. Such defects generally concern the positioning, assembly and/or shape of the various constituent elements of the article 2.

The processor 62 is also configured to collect and store in the memory 61 data with respect to the defects detected and articles 2 discarded due to the detection of such defects.

In the event that defects are detected in the sanitary absorbent articles 2 and/or abnormal operating parameters are detected, the processor 62 is configured to perform at least one of the following actions: generate an alarm or warning signal about the detected anomaly; generate a warning signal that the line 100 is producing potentially defective articles 2; generate a warning signal about the need to discard the articles 2 currently in production; automatically discard the articles 2 currently in production; automatically stop the production line 100 or at least one of the production devices 38, 40, 41, 42, 43.

In the case of discarding defective articles, the processor 62 is preferably configured to keep track in the memory 61 of the discard event that caused the discard and specific discard data indicative of the number of articles 2 discarded in association with that event.

Similarly, in the case of a production line stop, the processor 62 is preferably configured to keep track in the memory 61 of the stop event that caused the stop and specific stop data indicative of the stop time of the line 100 due to that event.

As mentioned above, in general, the production capacity of a plant can be measured through OEE, which is mainly derived from the three elements mentioned above: availability A, performance P and quality Q.

According to the invention, the processor 62 is configured to implement a method for controlling the production of absorbent sanitary articles 2 in the production line 100.

In particular, the control method of the invention is adapted to manage the OEE of the production line 100 by monitoring data related to the three elements of availability A, performance P and quality Q that affect OEE (specifically, discard event data, specific discard data, stop event data and specific stop data); processing such data so as to identify the root causes that may have had the greatest influence on the current OEE value of the production line 100 and identifying appropriate actions to be taken on the production line 100 so as to improve its OEE.

Relative to a determined time period of operation of the production line 100 (e.g. considering the work shifts in a month, a quarter, a semester or a calendar year), the processor 62 is configured to implement a method of controlling the production of absorbent sanitary articles 2 in the production line 100 that provides to:
- obtain discard event data indicative of discard events that led to discarding absorbent sanitary articles 2 during the determined time period and, for each discard event, specific discard data indicative of the number of absorbent sanitary articles discarded in association with the discard event; and
- obtain stop event data indicative of stop events that led to stop the production line 100 during the determined time period and, for each stop event, specific stop data indicative of a production line stop time associated with the stop event.

Discard event data, specific discard event data, stop event data and specific stop data can be retrieved from the memory 61 in which such events were previously collected and stored thanks to the action of the control system 60 and, in particular, thanks to the various operations described above carried out by the various components of said control system 60 (sensors 66, processor 62, emergency stop device 67, inspection device 72).

Discard events may include discard events of articles 2 due to the detection of a specific defect by the inspection device 72.

In addition, discard events of articles 2 may be related to the start or stop of the production line 100. It is noted, in this regard, that it is preferable to discard a pre-determined number of articles 2 at each re-start of the production line 100 in order to ensure that, in the event of a stop and re-start of the line 100 due to a malfunction or breakdown, the first articles 2 in output from the production line 100 to a downstream station (e.g. a packaging station, not shown, that packages the articles 2 in appropriate packaging) are up to standard (and not defective). It is also preferable to discard a predetermined number of articles 2 during each stop operation of the production line 100 in order to prevent that, while the production speed of the production line 100 is slowing down, the articles 2 are supplied to a downstream station of the production line 100 (e.g. a packaging station, not shown, that can pack the articles 2 in appropriate packages) at a speed that is too low and incompatible with the work rates of the downstream station.

Discard events of the articles 2 can also be associated with a fault found in the station (not shown) downstream of the production line 100. In fact, in such cases, when the downstream station is stopped or temporarily malfunctioning, it is customary to immediately discard the articles 2 produced by the production line 100 to avoid flooding the station downstream.

In turn, stop events may comprise stop events triggered automatically by the control system 60 due to the detection of a specific anomaly detected by the control system 60 (e.g., breakage or exhaustion or jamming of a material, malfunction or failure of a device etc. etc.) or after a certain number of discarded items 2 or due to a problem at the station downstream of the production line 100. Stop events may also comprise stop events triggered manually by an operator using the emergency stop device 67.

According to the method of the invention, once the aforementioned data has been obtained, the processor 62 is configured to:
- process the discard event data and the specific discard data obtained in order to classify the discard events according to their incidence on the total number of absorbent sanitary articles discarded during said determined time period and, from the classified discard events, select a limited number of discard events that had the greatest impact on the total number of discarded absorbent sanitary articles;
- process the stop event data and the specific stop data obtained in order to classify the stop events according to their incidence on a total stop time of the production line during said determined time period and, from the classified discard events, select a limited number of stop events that had the greatest impact on the total stop time of the production line;
- identify discard root causes that are respectively responsible for the selected limited number of discard events;
- identify stop root causes that are respectively responsible for the selected limited number of stop events.

Preferably, according to the Pareto principle, among the classified discard events, the limited number of discard events is selected by identifying the discard events that impacted for at least 80% on the total number of the discarded absorbent sanitary articles.

Preferably, according to the Pareto principle, from among the classified stop events, the limited number of stop events is selected by identifying the stop events that impacted for at least 80% on the total stop time of the production line.

The aforementioned classification of discard events and stop events and the subsequent identification of a limited number of stop events and discard events makes it advantageous to limit the identification of discard root causes and stop root causes to the limited number of identified stop events and discard events.

The Applicant observes that given the high construction complexity of the production line 100 and the high structural complexity of the articles 2 themselves, a discard event or stop event could be generated by several possible root causes. Tracing back, from among a plurality of possible root causes, to the root cause that is currently responsible for the discard event or stop event can be a non-immediate and complex operation. Consider, for example, a stop event due to an alarm generated by the identification of an abnormal operating parameter related to an irregular vibration of a device. This anomaly could be generated by different root causes such as, for example, the wear or breakage of a piece of the device itself, the malfunctioning of upstream devices that have caused the presence in the article of a weld or of a material with a thickness greater or smaller than expected, the blockage or the misalignment of a piece of the device itself and the like.

Limiting the root cause identification operation to the limited number of stop and discard events identified as described above advantageously makes the method of controlling the invention more efficient.

According to the invention, the stop root causes and discard root causes are identified by means of decision algorithms that allow - without the intervention of skilled operators - to automatically trace back, among a plurality of possible root causes, to the one that currently caused the stop event or discard event in question.

In particular, the stop root causes and the discard root causes are identified by means of decision algorithms based on at least one of the following items of information stored in the memory 61: known and/or self-learned data relating to a correlation between the discard events and a plurality of possible discard root causes; known and/or self-learned data relating to a correlation between the stop events and a plurality of possible stop root causes; current and/or historical information automatically provided by the control and monitoring system 60 of the production line 100 in association with the discard events and/or the stop events; current and/or historical information provided manually (as described above) by an operator at the control and monitoring system 60 in association with the discard events and/or the stop events; known and/or self-learned logic rules.

The aforesaid decision algorithms can be implemented by means of techniques known in the art that make use of artificial intelligence, expert system or preset and defined logics as well as a modelling based on a study of the stop events and discard events and the respective root causes most frequently found in production lines 100 of absorbent sanitary articles 2.

The decision algorithms can be assisted by special statistical algorithms and special machine learning algorithms.

Preferably, the processor 62 is configured to store in the memory 61 each currently identified stop/discard root cause in association with the respective stop/discard event in order to enrich the self-learned data on correlations between stop/discard events and possible stop/discharge root causes.

The identified root cause may relate to one or more of the production devices 38, 40, 41, 42, 43 and/or one or more of the accessory devices 45, which may be different from the device for which an anomalous operating parameter was detected by the sensors 65.

Discard root causes and stop root causes may for example comprise at least one cause selected from among: malfunctioning of a production device 38, 40, 41, 42, 43 or of an accessory device 45 of the production line 100, exhaustion of a material, defective material, stop of a work station downstream of the production line (e.g. packaging station), non-routine maintenance of the production line, absence of workforce.

For example, cases of stops that had the greatest impact on the total stop of the production line 100 include the case of a stop event originating from a weight sensor (such as, for example, a load cell) which, as a sensor 65 associated with the first conveyor belt 48, detects an absence of material deposited on the conveying surface 47 at one of the feeding devices 40. The processor 62 - by means of the aforementioned decision-making algorithms - can identify a material jam at the feeding device 40 as the root cause currently responsible for such a stop event, among other possible root causes such as, for example, a problem of material exhaustion or breakage or wear of a part of the feeding device 40.

A similar situation can occur, for example, in the case where a weight sensor (such as, for example, a load cell), as a sensor 65 associated with the first conveyor belt 48, detects an absence of elastic flaps 24 placed on the transport surface 47 at one of the feeding devices 40. In this case, the processor 62- by means of the aforesaid decision algorithms- can identify, as a root cause currently responsible for the absence of elastic flaps 24 which generated a stop or a discard event, a malfunctioning of the respective cutting device 43 adapted to cut a starting material to make the individual flaps 24, among other possible root causes such as, for example, a jamming of material at the respective feeding device 40 or a wrong feeding speed of the feeding device or a breakage or wear of a piece of the feeding device 40.

Consider also the situation of a glue dispensing device in one of the fixing devices 42 which is provided in its inside with a small secondary glue tank adapted to withdraw the glue, by means of a suitable withdrawal mechanism, from an external primary glue tank. In the event that a sensor 65 associated with the secondary glue tank detects that said tank is empty, the processor 62- by means of the aforesaid decision algorithms- can identify, as a root cause currently responsible for the empty secondary tank, a problem in the glue withdrawal mechanism, among other possible root causes such as, for example, an emptying of the primary glue tank.

In the aforesaid situation, also consider the case of a temperature sensor as a sensor 65 associated with the secondary glue tank of the glue dispensing device in one of the fixing devices 42. In the event that this temperature sensor detects an anomalous temperature value, the processor 62- -by means of the aforesaid decision algorithms - can identify, as a root cause currently responsible for the anomalous temperature value, a breakage or malfunctioning of the heating device (for example a coil) associated with the secondary glue tank, among other possible root causes such as, for example, the presence of an inadequate quantity of glue in the secondary glue tank.

In addition, in the event that a frequency meter as a sensor 65 associated with an ultrasonic fixing device 42 detects an anomalous working frequency, the processor 62 - by means of the aforesaid decision algorithms - can identify, as a malfunctioning cause currently responsible for the anomalous working frequency, a problem of misalignment between two welding heads (sonotrode and anvil) of the fixing device 42, among other possible root causes such as, for example, a problem of wear or breakage or malfunctioning or wrong setting of the sonotrode or of the fixing device 42 in general.

Once the stop root causes and discard root causes have been identified as described above, the processor 62 is configured, according to the method of the invention, to define an action to be implemented to remedy at least one of the identified stop root causes of detention and the identified discard root causes.

Preferably, the processor 62 is configured to automatically implement the defined action, which may consist of the automatic emission of a predetermined signal to an operator and/or a predetermined control signal for one or more of the actuators 66.

The warning to the operator may, for example, be an alarm or warning signal and comprise indications of the identified root cause and, possibly, a sequence of step-by-step instructions to guide the operator in implementing a corrective action to remedy the identified root cause.

Alternatively or additionally, sending the predetermined control signal to one or more of the actuators 66 enables them to implement in an automated manner at least part of the corrective intervention by acting on the production devices 38, 40, 41, 42, 43 and/or on the accessory devices 45 so as to remedy the original cause identified and, if necessary, bring the anomalous operating parameters detected by the sensors 65 back into compliance with the respective reference values.

For example, consider the case where a flow meter, as a sensor 65 associated with one of the fixing devices 42, detects a flow of dispensed glue that is outside respective reference values, the processor 62 can - by means of the aforesaid decision algorithms - identify as a root cause responsible for the anomalous flow of glue, an inadequate temperature and/or humidity value within the plant that houses the production line 100, among other possible root causes such as, for example, a breakage or malfunctioning of the heating device associated with a glue tank associated with the fixing device 42. In this case, the processor 62 may define a corrective action adapted to intervene on a temperature and/or humidity regulating device as actuator 66 so that it appropriately regulates the temperature and/or humidity level within the plant so as to bring the flow of dispensed glue back in compliance with the respective reference values.

A similar situation may occur, for example, in the case where a speed sensor, as a sensor 65 associated with one of the feeding devices 40, detects a material (e.g. cellulose fibre) feeding speed that is outside respective reference values. In this case, the processor 62 can identify as a root cause, responsible for the anomalous feeding speed, an inadequate humidity value within the plant that houses the production line 100, among other possible root causes such as, for example, a breakage or malfunctioning of the feeding device 40. Furthermore, the processor 62 can define a corrective action adapted to intervene on a humidity regulating device as actuator 66 so that it appropriately regulates the humidity level within the plant so as to bring the material feeding speed back in compliance with the respective reference values.

In another example, an optical sensor, as a sensor 65 associated with the transport members 38, may detect an inadequate level of cleanliness on the supporting surface 47. In this case, the processor 62 can identify as a root cause, responsible for the inadequate level of cleanliness, an anomalous temperature value, which is outside respective reference values, at the glue tank in one of the fixing devices 42, among other possible root causes such as, for example, a breakage or malfunctioning at one of the feeding devices 40. Furthermore, the processor 62 can define a corrective action adapted to intervene on a temperature regulating device as actuator 66 so that it appropriately regulates the temperature level inside the glue tank so as to bring the level of cleanliness on the supporting surface 47 back in compliance with the respective reference values.

With an appropriate feedback system, the processor 62 can check that the corrective actions taken are actually effective in bringing the anomalous operating parameters back in compliance with the respective reference values.

In particular, according to the method of the invention, the processor 62 is configured to determine an OEE index of the production line with respect to said determined time period and to determine it again for a further determined time period after implementation of the defined action.

The OEE index is determined by the processor 62 by obtaining from the automated control and monitoring system 60 of the production line 100, in relation to said determined time period:
- availability data A indicative of an actual operating time of the production line 100 with respect to a theoretical operating time,
- performance data P indicative of a quantity of absorbent sanitary articles 2 actually produced with respect to a theoretical expected quantity for the actual operating time of the line 100, and
- quality data Q relating to a quantity of absorbent sanitary articles 2 conforming to the quantity of absorbent sanitary articles 2 actually produced.

Afterwards, the two determined OEE values are compared with each other in order to verify the effectiveness of the actions taken on the OEE index.

The invention as a whole thus makes it possible to carry out an automated control of the production line 100 of absorbent sanitary articles 2 and, in particular, to manage its production efficiency by monitoring data that influence the trend of the OEE index and implementing corrective actions to improve it.

In particular, the control method of the invention is adapted to manage the OEE of the production line 100 by monitoring data related to the three elements of availability A, performance P and quality Q that affect OEE; processing such data so as to identify only the root causes that may have had the greatest influence on the current OEE value of the production line 100 and identifying appropriate actions to be taken on the production line 100 so as to improve its OEE. The data monitored are discard event data, specific discard data, stop event data and specific stop data.

By limiting the root cause identification and corrective actions to be implemented to the limited number of stop and discard events identified as described above, it is advantageously possible to manage the production effectiveness of the production line in an efficient and automated manner.

## Claims

1. Production line (100) for absorbent sanitary articles (2) comprising a working area (110) configured to carry out respective production operations on the absorbent sanitary articles (2) being processed along the production line (100) and an automated control and monitoring system (60) for the production line (100), the control and monitoring system (60) comprising control devices (64) operatively associated with the working area (110) and a central control unit (63) operatively connected to the control devices (64), the control unit (63) comprising a processor (62) which is configured, in relation to a determined time period of operation of the production line (100), to:
- obtain discard event data indicative of discard events that led to discarding absorbent sanitary articles (2) during the determined time period and, for each discard event, specific discard data indicative of the number of absorbent sanitary articles (2) discarded in association with the discard event;
- obtain stop event data indicative of stop events that led to stop the production line (100) during the determined time period and, for each stop event, specific stop data indicative of a stop time of the production line (100) associated with the stop event;
- process the discard event data and the specific discard data obtained in order to classify the discard events according to their incidence on the total number of absorbent sanitary articles (2) discarded during said determined time period and select a limited number of discard events that had the greatest impact on the total number of discarded absorbent sanitary articles (2);
- process the stop event data and the specific stop data obtained in order to classify the stop events according to their incidence on a total stop time of the production line (100) during said determined time period and select a limited number of stop events that had the greatest impact on the total stop time of the production line (100);
- identify discard root causes for the selected limited number of discard events;
- identify stop root causes for the selected limited number of stop events;
- define an action to be implemented with reference to at least one of the identified stop root causes and the identified discard root causes.

2. Production line (100) according to claim 1, wherein said processor (62) is configured to automatically implement the defined action, said action comprising automatically issuing a predetermined signal for an operator and/or a predetermined command signal for the control devices (64).

3. Production line (100) according to any one of the previous claims, wherein the control devices (64) comprise sensors (65) and wherein the processor (62) is configured to obtain discard event data, stop event data, discard specific data and stop specific data at least in part by processing data from said sensors (65).

4. Production line (100) according to any one of the previous claims, wherein the control devices (64) comprise at least one inspection device (72) configured to acquire article data related to the absorbent sanitary articles (2) being processed along the production line (100) and/or to the finished absorbent sanitary articles (2), in output from the production line (100), and wherein the processor (62) is configured to detect any defects on the absorbent sanitary articles (2) by processing the article data acquired by said at least one inspection device (72).

5. Production line (100) according to claim 4, wherein the processor (62) is configured to obtain discard event data, stop event data, specific discard data and specific stop data at least in part by processing data from said at least one inspection device (72).

6. Method of controlling the production of absorbent sanitary articles (2) in a production line (100), comprising, in relation to a determined time period of operation of the production line (100), the following steps:
- obtaining discard event data indicative of discard events that led to discarding absorbent sanitary articles (2) during the determined time period and, for each discard event, specific discard data indicative of the number of absorbent sanitary articles (2) discarded in association with the discard event;
- obtaining stop event data indicative of stop events that led to stop the production line (100) during the determined time period and, for each stop event, specific stop data indicative of a stop time of the production line (100) associated with the stop event;
- processing the discard event data and the specific discard data obtained in order to classify the discard events according to their impact on the total number of absorbent sanitary articles (2) discarded during the determined time period and selecting a limited number of discard events that had the greatest impact on the total number of discarded absorbent sanitary articles (2);
- processing the stop event data and the specific stop data obtained in order to classify the stop events according to their incidence on the total stop time of the production line during that specific time period and selecting a limited number of stop events that had the greatest impact on the total stop time of the production line (100);
- identifying discard root causes for the selected limited number of discard events;
- identifying stop root causes for the selected limited number of stop events;
- defining an action to be implemented with reference to at least one of the identified stop root causes and the identified discard root causes.

7. Method according to claim 6, comprising the step of automatically implementing the defined action, said action comprising automatically issuing a predetermined signal for an operator and/or a control signal for a control device (64) of the production line (100).

8. Method according to claim 6 or 7, wherein the stop root causes and the discard root causes are identified by decision algorithms based on at least one of the following information: known and/or self-learned data relating to a correlation between the discard events and a plurality of possible discard root causes; known and/or self-learned data relating to a correlation between the stop events and a plurality of possible stop root causes; current and/or historical information automatically provided by a control and monitoring system of the production line (100) in association with the discard events and/or the stop events; current and/or historical information provided by an operator in association with the discard events and/or the stop events; known and/or self-learned logic rules.

9. Method according to any one of claims 6 to 8, wherein the discard root causes and the stop root causes comprise respectively at least one discard root cause and at least one stop root cause selected from: malfunctioning of a production device (38, 40, 41, 42, 43) or other accessory device (45) of the production line (100), material exhaustion, defective material, stop of a workstation downstream of the production line (100), non-routine maintenance of the production line (100), absence of workforce.

10. Method according to any one of claims 6 to 9, wherein:
- among the classified discard events, the limited number of discard events is selected by identifying the discard events that impacted for at least 80% on the total number of the discarded absorbent sanitary articles (2); and
- among the classified stop events, the limited number of stop events is selected by identifying the stop events that impacted for at least 80% on the total stop time of the production line (100).

11. Method according to any one of claims 6 to 10, further comprising the steps of:
- obtaining, in relation to said determined time period, availability data A indicative of an actual operation time of the production line (100) with respect to a theoretical operation time, performance data P indicative of a quantity of absorbent sanitary articles (2) actually produced with respect to a theoretical expected quantity for the actual operation time of the production line (100) and quality data Q relating to a quantity of compliant absorbent sanitary articles (2) with respect to the quantity of absorbent sanitary articles (2) actually produced; and
- determining, in relation to said determined time period, an OEE index of the production line (100) based on the availability data A, the performance data P and the quality data Q obtained.

12. Method according to claims 7 and 11, further comprising the step of determining the OEE index in relation to a further determined time period after implementation of the defined action in order to verify the effectiveness thereof on the OEE index.

13. Method according to claim 11 or 12, wherein the availability data A, the performance data P and the quality data Q are obtained from an automated control and monitoring system (60) of the production line (100).

14. Method according to any one of claims 6 to 13, wherein the discard event data, the stop event data, the specific discard data and the specific stop data are obtained by an automated control and monitoring system (60) of the production line (100).
